# EUROPEAN PATENT SPECIFICATION

(11) **EP 2 520 182 B1**
(45) Date of publication and mention of the grant of the patent: **24.09.2014**
(21) Application number: 12166593.9
(22) Date of filing: 03.05.2012
(51) Int. Cl.: A23L 1/30, A61K 8/97, A61K 36/00

(54) **Plant composition comprising the phytocomplex of a plant species and process for preparing same**
Pflanzenzusammensetzung mit dem Phytokomplex einer Pflanzenart und Verfahren zu deren Herstellung
Composition végétale comprenant le phytocomplexe d'une espèce végétale et son procédé de préparation

(30) Priority: 03.05.2011 IT TO20110390
(43) Date of publication of application: 07.11.2012
(73) Proprietor: Daghero, Paolo, 10094 Giaveno (Torino) (IT); Cravotto, Giancarlo, 10137 Torino (IT)
(72) Inventor: Daghero, Paolo, 10094 Giaveno (Torino) (IT); Cravotto, Giancarlo, 10137 Torino (IT)
(74) Representative: Rambelli, Paolo

(56) References cited:
- EP-A2- 0 294 177
- EP-A2- 2 080 436
- FR-A1- 2 865 652
- DATABASE CAPLUS, [Online] 30 August 2006 (2006-08-30), XIE JUN ET AL: "METHOD FOR EXTRACTING POLYPHENOLS FROM PLANT", XP002497444, retrieved from CAPLUS Database accession no. 2006-901055
- CRAVOTTO G ET AL: "Improved extraction of vegetable oils under high-intensity ultrasound and/or microwaves", ULTRASONICS: SONOCHEMISTRY, BUTTERWORTH-HEINEMANN, GB, vol. 15, no. 5, 1 July 2008 (2008-07-01), pages 898-902, XP022594123, ISSN: 1350-4177 [retrieved on 2007-11-09]
- VILKHU ET AL: "Applications and opportunities for ultrasound assisted extraction in the food industry - A review", INNOVATIVE FOOD SCIENCE AND EMERGING TECHNOLOGIES, ELSEVIER, AMSTERDAM, NL, vol. 9, no. 2, 24 October 2007 (2007-10-24), pages 161-169, XP022476139, ISSN: 1466-8564, DOI: 10.1016/J.IFSET.2007.04.014

## Description

The present invention relates to a natural plant composition, comprising the phytocomplex of one or more plant species for use in the area of nutrition, in particular as a dietary supplement or for formulating dietary supplements or for phytotherapeutic or cosmetic use.

The term "phytocomplex" means in general all of the active principles, i.e. the pharmacologically active substances and the adjuvant principles, i.e. substances that are pharmacologically inactive in themselves, but contribute to producing the action of the active principles present in a plant species.

In practice, a phytocomplex contains, as active substances, glucosides, alkaloids, essential oils, tannins, flavonoids, vitamins and, as adjuvants, substances such as enzymes, amides, triglycerides, waxes, phospholipids, mineral salts, and resins. The bioavailability and pharmacological-therapeutic action of a plant is in general the resultant of the entire action of all the substances present in the phytocomplex.

The conventional extractive techniques used for preparing standardized plant extracts are laborious and costly in terms of time and material; these techniques involve the use of large amounts of solvents and can cause degradation of the desired molecules as well as partial loss of the components that are more volatile or have low solubility in the solvent used. The chemical characteristics of the extract are in fact highly dependent on the solvent used.

As is well known, the most recent developments in extraction techniques largely focus on finding solutions that minimize or avoid the use of solvents. Extractive techniques based on the use of ultrasound (Ultrasound-Assisted Extraction, UAE), extractive techniques aided by microwaves (Microwave-Assisted Extraction, MAE), or also techniques based on the combined use of ultrasound/microwaves, are of appreciable industrial interest.

The main advantages of these techniques are higher yield, the selectivity, stability and quality of the components present in the extracts, as well as a considerable reduction in extraction time.

It was recently demonstrated that the application of ultrasound improves the extraction of plant materials, mainly when carried out in such a way as to give rise to powerful acoustic cavitation. The mechanical effect of ultrasound accelerates the release of the organic compounds contained in the plant material, destroying the cell walls, increasing mass transfer and facilitating access of the solvent or of any type of fluid to the contents of the plant cells.

EP 2080435 A discloses the preparation of a plant extract from plant material by contacting the plant material and a solvent, extracting the active ingredients which are fixed on the exhausted plant material. The extraction step is carried out: under heating, preferably in the microwave, and under reduced pressure; under inert gas, preferably under nitrogen gas.

Extraction of plant phytocomplex with ultrasonic means are already disclosed in CAPLUS, 30 August 2006, XIE JUN ET AL: METHOD FOR EXTRACTING POLYPHENOLS FROM PLANT", accession no. 2006-901055 and in VILKHU ET AL: "Applications and opportunities for ultrasound assisted extraction in the food industry - A review", INNOVATIVE FOOD SCIENCE AND EMERGING TECHNOLOGIES, ELSEVIER, AMSTERDAM, NL, vol. 9, no. 2, 24 October 2007, pages 161-169.

One aim of the present invention is to provide a plant composition that allows the complete phytocomplex of a plant species to be made available.

Another aim of the invention is to provide a composition that improves the bioavailability of the active principles contained in plant species for use as a dietary supplement, in phytotherapy and in cosmetic applications.

Another aim of the invention is to provide a plant composition that achieves the aforementioned aims and that is completely natural, and does not contain vehicles, excipients or synthetic additives, or those extraneous to the plant species of interest.

In view of these aims, the invention relates to a plant composition and a process for preparation thereof, having the features defined in the claims given hereunder, which constitute an integral part of the present description.

The plant composition according to the invention, comprising the phytocomplex of one or more plant species, comprises the intracellular material enclosed within the cell wall of the species, obtainable by dispersion of particulate plant material in a liquid medium and ultrasonication treatment in conditions capable of destroying the cell wall to cause release of the aforementioned intracellular material in the liquid medium and further comprises the exhausted solid plant residue obtainable by the aforementioned ultrasonication treatment, where the intracellular material is absorbed on the exhausted solid plant residue and is present in the composition in an amount such that its weight ratio, on a dry basis, relative to the weight of the exhausted solid plant residue, is equal to at least 1.5 times the weight ratio of intracellular material to exhausted solid plant residue, obtainable by the aforementioned ultrasonication treatment, preferably in a ratio from about 2 times to about 3 times, in identical treatment conditions.

In other words, the plant composition according to the invention is a composition which is enriched of intracellular material relative to the amount of intracellular material of the plant species used and comprises an amount of the aforementioned intracellular material greater than or equal to at least 1.5 times, preferably from 3 to 8 times, the amount of intracellular material present in the species and obtainable by means of the ultrasonication treatment.

Enrichment, in the proportions indicated above, can be obtained by adding, to the product resulting from the ultrasonication treatment, the intracellular material obtained and separated from the exhausted solid plant residue in a prior ultrasonication treatment and submitting the enriched mixture thus obtained to drying, as described hereunder. Alternatively, enrichment can be obtained by carrying out a separation from the ultrasonication product of the exhausted solid plant residue and adding to the liquid suspension impoverished of the exhausted solid plant residue, an amount of exhausted solid plant residue metered in order to achieve the proportions stated above.

The term "exhausted solid plant residue" means the material derived from cell walls whose composition varies between different plant species and can also vary in relation to the state of growth of the plant. It mainly consists of insoluble fibres rich in cellulose, hemicellulose, pectin, lignin and some structural proteins (1-5%). Observation of this material under the microscope following the sonication treatment according to the invention identifies the presence of large pores due to the intense acoustic cavitation and to the violent collapse of gas bubbles.

The term intracellular material generally indicates the material enclosed within the cell wall, including cytoplasm, vacuoles, glands, membranes, etc.

Preferably, the composition further comprises the whole plant material of the plant species used. This plant material is used in dry, powdered form, preferably with particle size below 350 µm (for example 250-300 µm); the fraction of whole plant material can constitute from 0.5 to 35 wt.% of the composition, more preferably from about 5 to about 30 wt.%.

In the method of preparation, the liquid medium used in the ultrasonication step is typically water or an alcoholic or aqueous-alcoholic solution and can be selected in relation to the intended use of the plant composition according to the invention; a purely aqueous medium being preferred in the case of compositions for food and cosmetic use.

The ultrasonication stage is carried out in order to cause a strong cavitation effect. The resultant mechanical action causes disruption of the cell walls, a phenomenon that is much more pronounced at low frequencies (for example 18-30 kHz) but is practically negligible at high frequencies, for example between 200 kHz and 1000 kHz. Preferably, the sonication treatment therefore uses frequencies from 18 kHz to 45 kHz with power density in the range 50-500 W/100 mL, preferably from 100 to 500 or even more preferably from 250 to 500 W/100 mL.

Treatment of the powdered plant material, for example with particle sizes below 300 µm, in suspension with ultrasound gives rise to rapid destruction of the cell walls of the membranes. Ultrasonication produces cavitation effects when the acoustic power applied is high enough to permit the multiple production of microbubbles at the nucleation sites in the fluid. The bubbles grow during the rarefaction phase of the acoustic wave and then collapse during the compression phase. As a result of the collapse, a violent shock wave passes through the medium. The collapse of the bubbles converts the acoustic energy to mechanical energy in the form of shock waves equivalent to several thousand atmospheres of pressure (300 MPa).

This energy imparts motion to parts of cells, which disintegrate when their content of kinetic energy exceeds the strength of the cell wall. A further factor that increases the disruption of the cells is the phenomenon of microstreaming, due to high velocity gradients that cause shearing stresses that occur in the vicinity of gas bubbles, which vibrate radially owing to the ultrasound. Much of the energy absorbed by the cellular suspension is converted to heat, so that cooling is required during sonication.

As a result of the sonication treatment, the intracellular material, which is soluble, sparingly soluble and insoluble in the liquid medium used, is released into the liquid medium, so it is possible to carry out the ultrasonication stage using liquid media that do not have high solvent power with respect to the active principles that we wish to extract.

To avoid phenomena of oxidation of the active principles, such as enzymes for example, due to oxidation by free radicals, singlet oxygen and hydrogen peroxide, which may form in the sonication stage, it may be advantageous to introduce small amounts of antiradical agents or free radical scavengers, for example N₂O, into the liquid medium during the operation, for the purpose of reducing the inactivation of the aforementioned active principles.

The process can be carried out in batch mode; however, it is preferable to use continuous-flow sonication reactors, which have higher efficiency owing to the higher energy density and flexibility and hence lower energy consumption. An example of a pilot flow reactor usable in the process according to the invention is described in Cintas et al., Ultrason. Sonochem. 2010, 17(6), 985-989. This reactor has sonication chamber modules with volumes of about 0.5 ℓ fed in series. Typically, residence times in the sonication chamber from 15 seconds to 3 minutes can be used (for example 10-40 seconds).

The power density used is preferably between 200 and 500 W/100 mL.

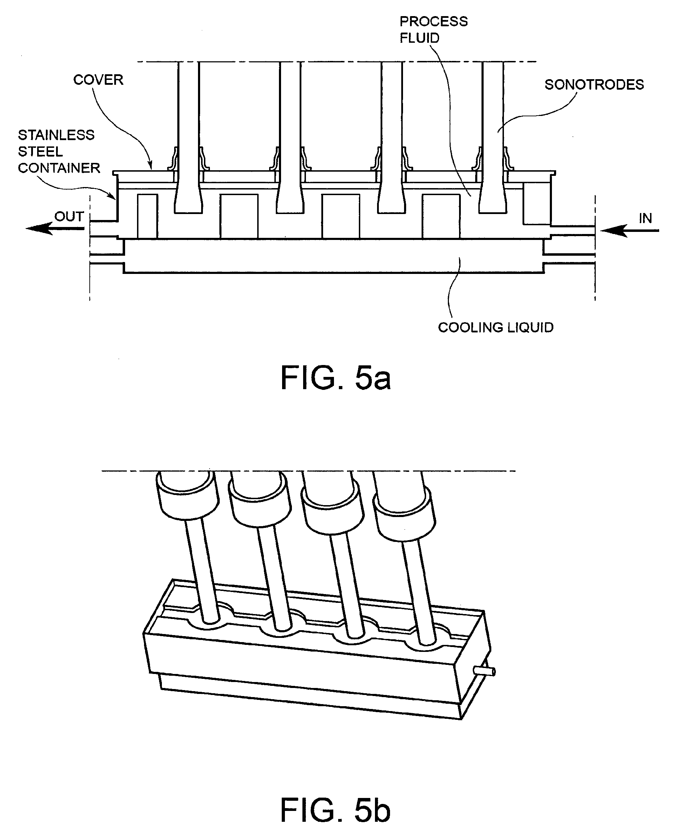

Preferably, a flow reactor is used, as shown in Fig. 5, arranged for minimizing the heating effect and optimizing the mechanical stress for destroying the cell wall or membranes and provided with a cooling jacket to maintain almost constant temperature of the suspension undergoing sonication.

To obtain the composition according to the invention, the suspension obtained by the sonication treatment, which is cloudy owing to the presence of the exhausted solid plant residue and the presence, in the liquid phase, of insoluble intracellular material, is then submitted to a stage of separation of the exhausted solid plant residue, for example by filtration or centrifugation.

Separation is preferably carried out in such a way as to maintain, in the liquid phase, insoluble intracellular material having micrometric dimensions, for example less than 20 µm.

The liquid phase containing the soluble and insoluble intracellular material of micrometric dimensions, following an optional stage of pasteurization and vacuum concentration, is submitted to evaporation by conventional techniques of spray drying, lyophilization or fluidized-bed drying. The aforementioned step of evaporation and drying is carried out by adding to the liquid suspension, after prior removal of the exhausted solid plant residue, an amount of exhausted solid plant residue, for example obtained from a preceding operation of ultrasonication and separation.

The amount of exhausted solid plant residue added in this step is such that the weight ratios given above are respected. It was found that the exhausted solid plant residue constitutes a matrix material of a filamentous nature, with a high specific surface, and constitutes an ideal support and excipient in the drying step. The high specific surface of this residual material means that the amounts of intracellular material that can be adsorbed on its surface are greater than those present in the original plant material.

The process according to the invention thus means that the drying step can be carried out without using excipients and supports that are extraneous to the plant species used; in particular, the composition according to the invention does not contain maltodextrins, which are typically used as excipient in the spray-drying step of plant extracts.

As already stated, the drying step can be carried out with addition of a fraction of the whole plant species, in powdered form, in the amounts stated above. Addition of the whole plant, in particular, results in supply of aromas or volatile components present in the fresh plant, obtaining a plant composition that also has the organoleptic characteristics corresponding to the fresh plant but in which the phytocomplex, adsorbed on the supporting plant material, is fully bioavailable.

The product obtained is in particulate form with particle size generally from 1 to 350 µm and has an extremely large surface area.

Further characteristics of the plant composition and of the process for preparation thereof will become clear from the examples given below, referring to the appended drawings, in which:

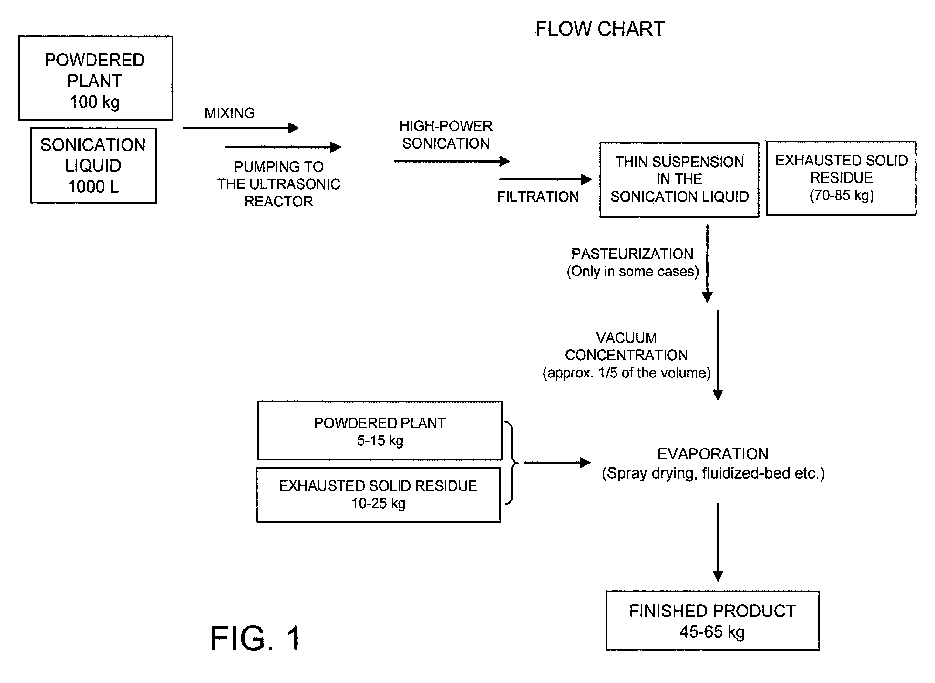

- Fig. 1 is a flow chart relating to a general example of carrying out the process;
- Fig. 2 is a ¹H NMR spectrum in DMSO-d6 of the composition obtained according to example 2 given hereunder, compared with a commercial extract;
- Fig. 3 is a ¹H NMR spectrum in DMSO-d6 of a composition obtained from artichoke, according to example 3, compared with the spectrum of a commercial composition, containing maltodextrins;
- Fig. 4 shows the ¹H NMR spectrum in DMSO-d6 of a composition according to the invention, obtained according to example 4, of *Harpagophytum procumbens* (devil's claw); and
- Figs. 5a and 5b show a schematic representation of a flow reactor usable for the process according to the invention and, respectively, a detail from Fig. 5a.

### Example 1

This example relates to the treatment of 100 kg of powdered plant; the amounts by weight shown, expressed in kg, are therefore also the percentages by weight relative to 100 parts by weight of material treated.

The powdered plant, previously dried, with particle size generally not above 300 µm, is dispersed in a stirred mixer in 1000 ℓ of sonication liquid (water, aqueous-alcoholic solution). After mixing, the suspension is fed by an ultrasonic reactor and submitted to high-power sonication.

The industrial-scale sonication treatment is preferably carried out in a continuous-flow reactor having the following characteristics: system with modules, or multi-probe sonication cells (from 2 to 10), mounted in series in order to ensure the necessary mechanical action depending on the physical characteristics of the plant material to be extracted.

At the end of sonication, the exhausted solid plant residue is separated from the suspension thus obtained; separation is preferably performed by filtration or by centrifugation, so as to maintain the soluble, insoluble and sparingly soluble intracellular components in the liquid medium.

Typically from 70 kg to 85 kg of exhausted solid plant residue is obtained (based on dry matter). The thin suspension, which is cloudy due to the presence of insoluble matter, can optionally undergo pasteurization and then, preferably, an operation of vacuum concentration in order to reduce the volume to about 40% to 90% of the initial volume (for example 1/5 of the initial volume).

A portion of the exhausted solid plant residue, obtained in a separation step or separated in a previous extraction, is added to the concentrated suspension thus obtained, which contains from 15 kg to 30 kg of the extracted material. In the example shown, from 15 kg to 25 kg of exhausted solid plant residue is added, relative to an amount from 30 kg to 15 kg of extraction product contained in the concentrated suspension, and the suspension thus obtained is submitted to evaporation, for example by spray drying, lyophilization or in a fluidized-bed reactor.

The weight ratio of plant substance extracted to exhausted solid plant residue, in the suspension fed to the evaporation stage, is then equal to 3.5-7.2 times and can be up to 8 times the corresponding weight ratio obtained as a result of the sonication treatment (in the same conditions). Optionally, a further 5 kg to 15 kg of powdered plant, with particle size below 300 µm and previously dried and optionally pasteurized, is added to the suspension fed to evaporation. From 45 kg to 65 kg of a composition according to the invention is obtained, in which the intracellular material extracted is adsorbed on the exhausted solid plant residue and optionally on the particulate material of the powdered plant.

The following examples relate to products obtained by sonication in laboratory sonication reactors.

### Example 2 - Guaraná (Paullinia cupana Kunth)

The process is carried out with the following parameters.

Feed: powdered plant seeds

Liquid medium: water

Plant/liquid medium ratio: 1:10

Sonication temperature: 45-48°C

Total volume of the solid-liquid suspension: about 3200 ml

A high-power ultrasonication reactor is used, with a 500-ml sonication chamber, into which a volume of 300 ml is fed.

Ultrasound power density: 100 W/50 ml

Residence time in the sonication chamber: 4 minutes, after which the suspension is filtered rapidly, maintaining the sonication conditions.

The filtrate is preconcentrated and spray-dried in a BÜCHI MINISPRAY DRYER B-290.

The exhausted solid plant residue (5-10% of the final product) and the whole plant powder (15-20% of the final composition) are added as adjuvants in the drying step.

Active principle determined: caffeine 10%.

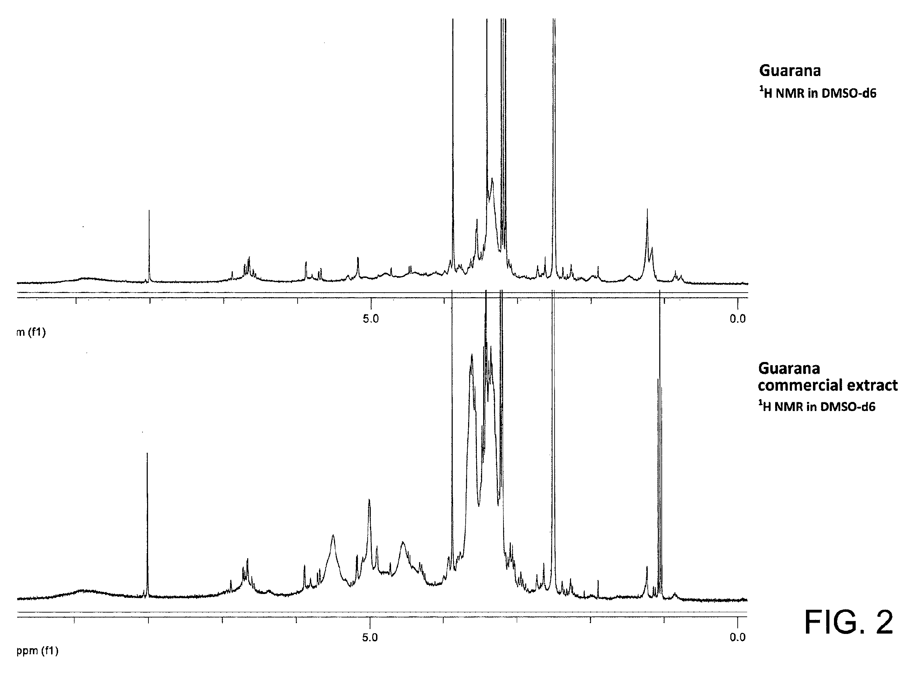

Fig. 2 shows the ¹H NMR spectrum in DMSO-d6 of the composition obtained, compared with that of a commercial extract.

### Example 3 - Artichoke (Cynara scolymus, Linn.)

Feed: powdered leaves

Sonication medium: water

Plant/medium ratio: 1:10

Sonication temperature: 45-48°C

Total volume of the solid-liquid suspension: about 3200 ml.

Sonication and spray drying are carried out in the same conditions as shown for example 2 with the same parameters and amounts added.

Active principle determined: caffeoylquinic acids, such as chlorogenic acid 6%.

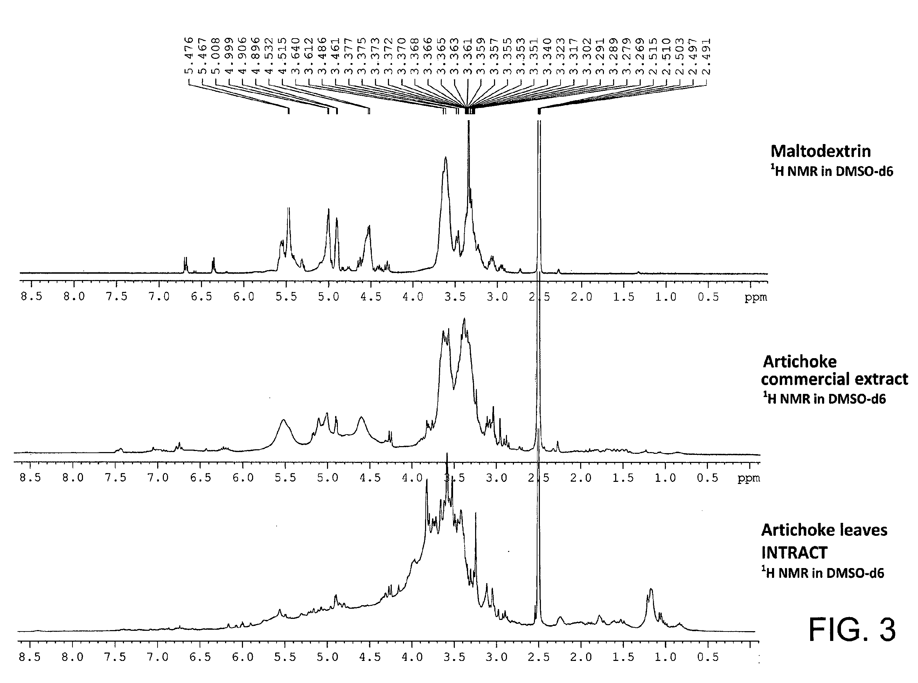

Fig. 3 shows the ¹H NMR spectrum in DMSO-d6 of the composition obtained compared with a commercial composition containing maltodextrins, and with the spectrum of maltodextrins.

### Example 4 - Devil's claw (Harpagophytum procumbens)

Feed: powdered roots

Sonication medium: water/ethanol in 8:2 ratio

Ratio of plant material to sonication medium: 1:12

Sonication temperature: 40-43°C

Total volume of the solid-liquid suspension: about 3200 ml.

A sonication reactor with a 500-ml sonication chamber is used, with feed of a volume of 300 ml.

Ultrasound power density: 100 W/50 ml

Residence time in the sonication chamber: 5 minutes, after which the suspension is filtered rapidly, with sonication.

The filtrate is preconcentrated and then spray-dried in a BÜCHI MINISPRAY DRYER B-290.

The exhausted solid plant residue (8-13% of the final composition) and plant material of the whole plant (15-20% of the final composition) are added as adjuvants in the spray-drying step.

Active principle determined: arpagoside 2.5%.

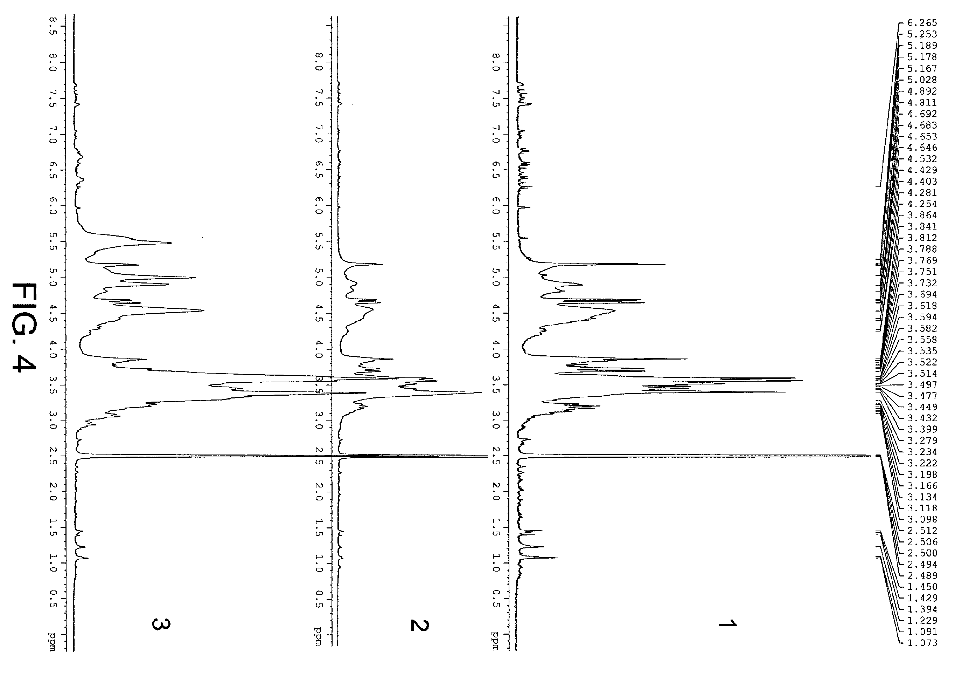

Fig. 4 shows the ¹H NMR spectrum in DMSO-d6 of the composition obtained, compared with a commercial extract containing maltodextrins.

The NMR analyses were performed by dissolving the samples in deuterated dimethylsulphoxide. The spectra were recorded on a Bruker Avance 300 spectrometer at 300 MHz (25°C). The chemical shifts (δ) are given in ppm and are calibrated for the particular solvent.

The analyses by TLC on the samples obtained show that compositions according to the invention show the same analytical composition of the whole phytocomplex detected in the plant powder. TLC analysis in fact shows perfect correspondence between the original composition of the plant and the composition according to the invention, as the profiles of the two spots in TLC are perfectly superimposable.

Further, non-limiting, examples of plant materials that can be submitted to the process for obtaining compositions according to the invention comprise: *Passiflora internata, Panax ginseng, Ilex paraguariensis* (maté), *Melissa officinalis, Betula alba, Taraxacum officinale, Cassia angustifoglia, Rosa canina L, Eleutherococcus senticosus, Eschscholzia californica.*

## Claims

1. Plant composition comprising the phytocomplex of a plant species, or a mixture of phytocomplexes of different plant species, **characterised in that** said plant composition comprises:
- the intracellular material of the plant species obtainable by dispersing plant material in particulate form in a liquid medium and by ultrasonic treatment in conditions that destroy the cell wall to cause the release of said intracellular material in the liquid medium; and
- the exhausted solid plant residue obtainable from the aforesaid ultrasonic treatment, **characterised in that** the intracellular material is absorbed on said exhausted solid plant residue and in which the weight ratio, on an anhydrous basis, between the intracellular material and the exhausted solid plant residue is at least 1.5 times the weight ratio between the intracellular material and the exhausted solid plant residue obtainable by the ultrasonic treatment.

2. Composition according to claim 1, **characterised in that** it also includes powdered whole material of the plant species, optionally pasteurized.

3. Composition according to claims 1 or 2, comprising from 5% to 30% by weight, referred to the weight of the composition, of powdered whole plant material.

4. Composition according to any of claims 1 to 3, wherein said ratio between intracellular material is from 3 to 8 times the weight ratio between intracellular material and exhausted solid plant residue obtainable from ultrasonic treatment.

5. Composition according to any of claims 1 to 4, **characterised in that** it is free of excipients, carriers and adjuvant foreign to the plant material from which the composition is obtained.

6. Composition according to any of claims 1 to 5, **characterised in that** it is free from maltodextrins.

7. Composition according to any of the preceding claims, **characterised in that** said intracellular material comprises intracellular material which is insoluble or poorly soluble in the liquid medium of sonication.

8. Process for producing a plant composition, comprising the phytocomplex of a plant species, or a mixture of phytocomplexes of different plant species comprising the steps of:
a) ultrasonic treatment of particulate plant material in a liquid medium, under conditions which destroy the cell wall and cause the release in the liquid medium of intracellular plant material, thereby to obtain a suspension comprising the exhausted solid plant residue derived from the destroyed cell wall and the said intracellular material soluble and insoluble in the liquid medium;
b) drying the intracellular plant material of step a) in the presence of the exhausted solid plant residue, so as to cause the absorption of the intracellular plant material on the exhausted solid plant residue, wherein in said drying step the weight ratio between intracellular plant material and exhausted solid plant residue is adjusted so as to be at least 1.5 times the weight ratio between said materials, as obtainable from step a).

9. Process according to claim 8, wherein the weight ratio between the intracellular plant material and exhausted solid plant residue is from 3 to 8 times the weight ratio between said materials, as obtainable from step a).

10. Process according to claims 8 or 9, **characterised in that** the drying process is carried out with addition of whole powdered plant material, optionally pasteurized, in an amount from 5 to 30% by weight referred to the weight of the final composition.

11. Process according to any one of claims 8 to 10, **characterised in that**, following step a), it comprises the step of separating from the suspension obtained in step a) the exhausted solid plant residue by filtration or centrifugation and the addition to the liquid suspension depleted of said exhausted solid plant residue, of a portion of exhausted solid plant residue dosed to achieve said weight ratio between intracellular material and exhausted solid plant residue and feeding the suspension thus obtained, optionally following concentration, to the drying stage.

12. Process according to any one of claims 8 to 11, in which the treatment of sonication is carried out by applying to the suspension a power density of 50 to 500 W/100 mL, preferably from 100 to 500.

13. Process according to any one of claims 8 to 12, wherein the sonication step is carried out in the presence of an anti-radical agent in the liquid medium.

## Patentansprüche

1. Pflanzenzusammensetzung, umfassend den Phytokomplex einer Pflanzenart oder eine Mischung aus Phytokomplexen verschiedener Pflanzenarten, **dadurch gekennzeichnet, dass** die Pflanzenzusammensetzung umfasst:
- das intrazelluläre Material der Pflanzenart, erhältlich durch Dispergieren von Pflanzen material in Partikelform in einem flüssigen Medium und durch Ultraschallbehandlung bei Bedingungen, welche die Zellwand zerstören, um die Freisetzung des intrazellulären Materials in das flüssige Medium zu bewirken, und
- die erschöpften festen Pflanzenreststoffe, erhältlich aus der vorstehend genannten Ultraschallbehandlung,
**dadurch gekennzeichnet, dass** das intrazelluläre Material auf den erschöpften festen Pflanzenreststoffen absorbiert ist und in dem das Gewichtsverhältnis auf wasserfreier Basis zwischen dem intrazellulären Material und den erschöpften festen Pflanzenreststoffen mindestens das 1,5-fache des Gewichtsverhältnisses zwischen dem durch die Ultraschallbehandlung erhältlichen intrazellulären Material und den erschöpften festen Pflanzenreststoffen beträgt.

2. Zusammensetzung nach Anspruch 1, **dadurch gekennzeichnet, dass** sie auch pulverisiertes gesamtes Material der Pflanzenart, das gegebenenfalls pasteurisiert ist, enthält.

3. Zusammensetzung nach Anspruch 1 oder 2, umfassend von 5 bis 30 Gew.-%, bezogen auf das Gewicht der Zusammensetzung, des pulverisierten gesamten Pflanzenmaterials.

4. Zusammensetzung nach einem der Ansprüche 1 bis 3, wobei das Verhältnis zwischen intrazellulärem Material das 3- bis 8-fache des Gewichtsverhältnisses zwischen aus der Ultraschallbehandlung erhältlichem intrazellulärem Material und erschöpften festen Pflanzenreststoffen beträgt.

5. Zusammensetzung nach einem der Ansprüche 1 bis 4, **dadurch gekennzeichnet, dass** sie frei von Hilfsstoffen, Trägern und Adjuvantien ist, welche dem Pflanzenmaterial, von dem die Zusammensetzung erhalten wird, fremd sind.

6. Zusammensetzung nach einem der Ansprüche 1 bis 5, **dadurch gekennzeichnet, dass** sie frei von Maltodextrinen ist.

7. Zusammensetzung nach einem der vorhergehenden Ansprüche, **dadurch gekennzeichnet, dass** das intrazelluläre Material intrazelluläres Material umfasst, welches in dem flüssigen Medium der Ultraschallbehandlung unlöslich oder schwach löslich ist.

8. Verfahren zur Herstellung einer Pflanzenzusammensetzung, umfassend den Phytokomplex einer Pflanzenart oder eine Mischung aus Phytokomplexen verschiedener Pflanzenarten, umfassend die Schritte:
a) Ultraschallbehandlung von partikelförmigem Pflanzenmaterial in einem flüssigen Medium bei Bedingungen, welche die Zellwand zerstören und die Freisetzung des intrazellulären Pflanzenmaterials in das flüssige Medium bewirken, um dadurch eine Suspension zu erhalten, umfassend die erschöpften festen Pflanzenreststoffe, die von der zerstörten Zellwand stammen, und das in dem flüssigen Medium lösliche und unlösliche intrazelluläre Material,
b) Trocknen des intrazellulären Pflanzenmaterials aus Schritt a) in Gegenwart der erschöpften festen Pflanzenreststoffe, um die Absorption des intrazellulären Pflanzenmaterials an die erschöpften festen Pflanzenreststoffe zu bewirken, wobei in dem Trocknungsschritt das Gewichtsverhältnis zwischen intrazellulärem Pflanzenmaterial und erschöpften festen Pflanzenreststoffen so eingestellt wird, dass es mindestens das 1,5-fache des Gewichtsverhältnisses zwischen den in Schritt a) erhaltenen Materialien beträgt.

9. Verfahren nach Anspruch 8, wobei das Gewichtsverhältnis zwischen dem intrazellulären Pflanzenmaterial und den erschöpften festen Pflanzenreststoffen das 3 bis 8-fache des Gewichtsverhältnisses zwischen den aus Schritt a) erhaltenen Materialien beträgt.

10. Verfahren nach Anspruch 8 oder 9, **dadurch gekennzeichnet, dass** das Trockungsverfahren mit Zugabe von gesamtem pulverisierten Pflanzenmaterial, das gegebenenfalls pasteurisiert ist, in einer Menge von 5 bis 30 Gew.-%, bezogen auf das Gewicht der finalen Zusammensetzung, durchgeführt wird.

11. Verfahren nach einem der Ansprüche 8 bis 10, **dadurch gekennzeichnet, dass** es, nach Schritt a), den Schritt des Trennens der erschöpften festen Pflanzenreststoffe von der in Schritt a) erhaltenen Suspension durch Filtration oder Zentrifugation, und die Zugabe eines Teils der erschöpften Festpflanzenreststoffe, die dosiert sind, um das Gewichtsverhältnis zwischen intrazellulärem Material und erschöpften festen Pflanzenreststoffen zu erreichen, zu der flüssigen Suspension, aus der die erschöpften festen Pflanzenreststoffe depletiert wurden, und das Zuführen der so erhaltenen Suspension, gegebenenfalls nach Konzentration, an den Trocknungsschritt, umfasst

12. Verfahren nach einem der Ansprüche 8 bis 11, in dem die Ultraschallbehandlung durch Anwenden einer Leistungsdichte von 50 bis 500 W/100 mL, vorzugsweise von 100 bis 500, auf die Suspension durchgeführt wird.

13. Verfahren nach einem der Ansprüche 8 bis 12, wobei der Ultraschallbehandlungsschritt in Gegenwart eines Anti-Radikalmittels in dem flüssigen Medium durchgeführt wird.

## Revendications

1. Composition végétale comprenant le phytocomplexe d'une espèce végétale ou un mélange de phytocomplexes de différentes espèces végétales, **caractérisée en ce que** ladite composition végétale comprend :
- la matière intracellulaire de l'espèce végétale, accessible par dispersion du matériau végétal sous forme de particules dans un milieu liquide et par traitement aux ultrasons dans des conditions qui font que les parois cellulaires sont détruites et que ladite matière intracellulaire est libérée dans le milieu liquide,
- et le résidu végétal solide épuisé, obtenu à l'issue du traitement aux ultrasons mentionné ci-dessus ;
et **caractérisée en ce que** la matière intracellulaire est absorbée sur ledit résidu végétal solide épuisé, et dans laquelle le rapport pondéral à l'état anhydre entre la matière intracellulaire et ledit résidu végétal solide épuisé vaut au moins 1,5 fois le rapport pondéral accessible par le traitement aux ultrasons entre la matière intracellulaire et le résidu végétal solide épuisé.

2. Composition conforme à la revendication 1, **caractérisée en ce qu'**elle comprend aussi du matériau entier, réduit en poudre, de l'espèce végétale, en option pasteurisé.

3. Composition conforme à la revendication 1 ou 2, comprenant de 5 à 30 %, en poids rapporté au poids de la composition, de matériau végétal entier réduit en poudre.

4. Composition conforme à l'une des revendications 1 à 3, dans laquelle ledit rapport de matière intracellulaire vaut de 3 à 8 fois le rapport pondéral accessible par traitement aux ultrasons entre la matière intracellulaire et le résidu végétal solide épuisé.

5. Composition conforme à l'une des revendications 1 à 4, **caractérisée en ce qu'**elle ne contient pas d'excipients, de véhicules ou d'adjuvants étrangers au matériau végétal à partir duquel est obtenue la composition.

6. Composition conforme à l'une des revendications 1 à 5, **caractérisée en ce qu'**elle ne contient pas de maltodextrines.

7. Composition conforme à l'une des revendications précédentes, **caractérisée en ce que** ladite matière intracelulaire comprend de la matière intracellulaire qui est insoluble ou peu soluble dans le milieu liquide de sonication.

8. Procédé de production d'une composition végétale comprenant le phytocomplexe d'une espèce végétale ou un mélange de phytocomplexes de différentes espèces végétales, lequel procédé comporte les étapes suivantes :
a) traiter aux ultrasons le matériau végétal, réduit en particules, dans un milieu liquide, dans des conditions qui font que les parois cellulaires sont détruites et que la matière végétale intracellulaire est libérée dans le milieu liquide, de manière à obtenir une suspension comprenant le résidu végétal solide épuisé, dérivé des parois cellulaires détruites, et les fractions soluble et insoluble dans le milieu liquide de ladite matière intracellulaire,
b) et faire sécher la matière végétale intracellulaire de l'étape (a), en présence du résidu végétal solide épuisé, de manière à ce que la matière végétale intracellulaire soit absorbée sur le résidu végétal solide épuisé, et dans lequel procédé le rapport pondéral entre la matière végétale intracellulaire et le résidu végétal solide épuisé est ajusté, dans ladite étape de séchage, de manière à ce que ce rapport vaille au moins 1,5 fois le rapport pondéral accessible à l'issue de l'étape (a) entre ces matériaux.

9. Procédé conforme à la revendication 8, dans lequel le rapport pondéral entre la matière végétale intracellulaire et le résidu végétal solide épuisé vaut de 3 à 8 fois le rapport pondéral accessible à l'issue de l'étape (a) entre ces matériaux.

10. Procédé conforme à la revendication 8 ou 9, **caractérisé en ce que**, lors de l'opération de séchage, on ajoute du matériau végétal entier réduit en poudre, en option pasteurisé, en une quantité représentant de 5 à 30 %, en poids rapporté au poids de la composition finale.

11. Procédé conforme à l'une des revendications 8 à 10, **caractérisé en ce qu'**il comporte, à la suite de l'étape (a), une étape au cours de laquelle on sépare de la suspension issue de cette étape (a) le résidu végétal solide épuisé, par filtration ou centrifugation, puis on ajoute, à la suspension liquide appauvrie en ledit résidu végétal solide épuisé, une portion de ce résidu végétal solide épuisé, dosée de manière à obtenir ledit rapport pondéral entre la matière intracellulaire et le résidu végétal solide épuisé, et l'on envoie la suspension ainsi obtenue, éventuellement après l'avoir concentrée, subir l'opération de séchage,

12. Procédé conforme à l'une des revendications 8 à 11, dans lequel on réalise le traitement de sonication en appliquant à la suspension une densité d'énergie de 50 à 500 W par 100 mL, et de préférence de 100 à 500 W par 100 mL.

13. Procédé conforme à l'une des revendications 8 à 12, dans lequel on effectue l'étape de sonication en présence, dans le milieu liquide, d'un agent anti-radicaux.
